**Europäisches Patentamt**

**European Patent Office**

**Office européen des brevets**

(19)

(11) Numéro de publication: **0 005 400**
**B1**

(12) **FASCICULE DE BREVET EUROPEEN**

(45) Date de publication du fascicule de brevet: **15.04.81**

(21) Numéro de dépôt: **79400276.6**

(22) Date de dépôt: **27.04.79**

(51) Int. Cl.³: **C 07 C 148/00,**
**C 07 C 149/06**

(54) **Procédé de synthèse de mercaptans.**

(30) Priorité: **05.05.78 FR 7813341**

(43) Date de publication de la demande:
**14.11.79 Bulletin 79/23**

(45) Mention de la délivrance du brevet:
**15.04.81 Bulletin 81/15**

(84) Etats Contractants Désignés:
**DE GB NL**

(56) Documents cités:
**FR - A - 2 333 006**
**FR - A - 2 333 784**
**US - A - 2 411 983**
**US - A - 3 682 804**

(73) Titulaire: **SOCIETE NATIONALE ELF AQUITAINE**
**Tour Aquitaine**
**F-92400 Courbevoie (FR)**

(72) Inventeur: **Ollivier, Jean**
**Croix de Buzy**
**F-64260 Arudy (FR)**
Inventeur: **Souloumiac, Guy**
**3 Allée Duke Ellington**
**F-64000 PAU (FR)**
Inventeur: **Suberlucq, Jeannine**
**28 rue Louis Lacaze**
**F-64000 Pau (FR)**

(74) Mandataire: **Kohn, Armand**
**5 Avenue Foch**
**F-92380 Garches (FR)**

Courier Press, Leamington Spa, England.

## 0 005 400

### Procédé de synthèse de mercaptans

La présente invention constitue un perfectionnement à la production de mercaptans à partir de composés éthyléniques, par la réaction photochimique de ces derniers avec de l'hydrogène sulfuré; elle vise, en particulier, la synthèse photochimique de mercaptans à partir d'oléfines et à partir d'esters éthyléniques, en phase liquide.

Etant donné l'utilité industrielle bien connue de différents mercaptans, les méthodes de préparation de ces corps ont donné lieu à de nombreux travaux; un procédé, qui a conduit à des résultats industriels intéressants, réside dans l'action directe de l'hydrogène sulfuré sur des substances éthyléniques, sous l'effet de radiations ultraviolettes. Dans la pratique de ce procédé, on a été conduit à introduire, dans le milieu réactionnel, des agents promoteurs, et en particulier des phosphites organiques, comme décrit, par exemple, dans le brevet US 3 050 452. La création photochimique des radicaux SH, devant induire l'addition de l'H$_2$S sur la double liaison du composé éthylénique, implique l'utilisation des radiations ultraviolettes de courtes longueurs d'onde, ce qui présente certains inconvénients. En effet, on est obligé d'utiliser des réactifs et des solvants particulièrement purs et transparents; d'autre part, le parcours optique dans le réacteur est faible à cause du coefficient d'extinction moléculaire assez fort de l'hydrogène sulfurè; en outre, le mercaptan synthétisè absorbe lui-même des radiations, et cela provoque un ralentissement considérable des réactions pour des degrés d'avancement peu élevés, avec formation de sulfure par addition du mercaptan lui-même sur la double liaison du composé éthylénique.

Pour éviter ces inconvénients et pour pouvoir mettre en oeuvre industriellement le procédé sus-indiqué, avec des vitesses de réaction et des rendements convenables, il faudrait donc pouvoir opérer l'addition photochimique dans un domaine de longueurs d'onde plus grandes, c'est-à-dire proches du spectre visible, ou éventuellement avec de la lumière visible.

La présente invention apporte justement une solution à ce probléme par l'application d'une substance sensibilisatrice, pouvant être excitée dans un domaine de longueurs d'onde au-dessus de 300 nm, alors que les seuls résultats industriels, obtenus jusqu'à présent, exigeaient des longueurs d'onde inférieures à 300 nm.

Le procédé suivant l'invention est caractérisé en ce que le milieu réactionnel habituel, contenant un promoteur constitué par un dérivé organique d'un élément du groupe V A de la Classification Périodique des Eléments, est additionné de benzophénone ou/et thio-benzophénone ou bien d'un ou de plusieurs dérivés de ces arylcétones.

Les adjuvants, suivant l'invention, permettent d'effectuer la synthèse photochimique des mercaptans dans un domaine de longueurs d'onde s'étendant depuis environ 300 nm jusqu'à 600 nm. En effet, l'adjonction de benzophénone, C$_6$H$_5$COC$_6$H$_5$, dont le spectre d'absorption du premier état excité, réactif, couvre la région de 300 à 400 nm, permet donc de travailler dans un ultraviolet trés voisin du spectre visible. La thiobenzophénone, C$_6$H$_5$CSC$_6$H$_5$, est active dans la région de 350 à 600 nm qui, — comme on le voit — empiète sur le domaine visible, puisque celui-ci s'étend de 400 à 750 nm. L'emploi simultané de la benzophénone avec la thiobenzophénone, en proportions appropriées, présente l'avantage de permettre le travail dans la zone de longueurs d'onde, très large, de 300 à 600 nm; on peut ainsi bénéficier au maximum de l'énergie émise par les générateurs de l'ultraviolet de puissance, actuellement disponibles sur le marché industriel.

Bien que la benzophénone et la thiobenzophénone non substituées donnent d'excellents résultats, il peut être bon d'employer certains de leurs dérivés, notamment des composés dans lesquels un ou les deux noyaux benzéniques portent des substituants tels que alkyles, halogènes, hydroxyles, groupes alkoxy ou esters.

Les promoteurs de l'art connu, les plus employés, sont les phosphites trialkyliques et triaryliques: les cétones et thiones, suivant la présente invention, donnent de bons résultats en conjonction avec eux, mais ces dérivés du phosphore peuvent être remplacés ou accompagnés de dérivés organiques de Bi, As, ou Sb.

Les adjuvants, suivant l'invention, permettent d'amplifier l'effet initiateur des agents connus, notamment des phosphites mentionnés plus haut, et de limiter l'action inhibitrice que les sulfures, éventuellement formés par une réaction secondaire, produisent sur la formation du mercaptan recherché.

Bien que les cétones et thiones aryliques, suivant l'invention, puissent être utilisées seules en tant qu'initiateurs photochimiques, dans le domaine des longueurs d'onde supérieures à 300 nm, elles donnent des résultats remarquablement meilleurs que les promoteurs connus, lorsqu'elles sont employées conjointement avec des composés organiques d'éléments du groupe VA de la classification périodique, en particulier phosphites trialkyliques et triaryliques.

Ainsi, par exemple, dans la synthèse du n-propanethiol à partir du propylène et de l'H$_2$S, en présence de l'ultraviolet de 350 nm, avec 0,06 mole de phosphite de tridécyle par litre de milieu réactionnel, on n'obtient qu'un peu plus de la moitié de la quantité de n-propanethiol que donne 0,014 mole de benzophénone; mais si l'on utilise, à la fois, les mêmes proportions de ces deux initiateurs, la quantité de mercaptan obtenu est environ 2,25 fois plus grande que celle à laquelle conduit le phosphite de tridécyle seul. On voit donc que les benzophénones et thiobenzophénones apportent un avantage très marqué sur les anciens initiateurs. Ces résultats sont d'autant plus inattendus qu'une cétone, notamment l'acétone, avait été proposée comme promoteur d'une réaction photochimique de certains mercaptans avec du polybutadiène (brevet US 3 338 810). Or, la présente invention montre que, contrairement à l'acétone, les benzophénones et thiobenzophénones ne présentent pas de réactivité chimique vis-à-vis de l'hydrogène sulfuré et du mercaptan formé.

Les proportions des initiateurs, suivant l'invention, à ajouter au milieu réactionnel, dépendent des différents facteurs, notamment de la nature des réactifs traités, et de leur concentration dans le milieu, de la présence ou de l'absence du solvant, etc. Le plus souvent, ces proportions sont de l'ordre de 0,001 à 0,05 mole par litre de milieu réactionnel, et surtout de 0,0015 à 0,0075.

Le procédé de l'invention est, de préférence, réalisé au sein d'un solvant qui peut être par exemple un hydrocarbure ou un éther, mais non pas un sulfure. Conviennent, en particulier des solvants tels que méthylal, glyoxal, éther diméthylique de l'éthylène glycol, diméthyl-éther du diéthylène glycol, benzène, toluène, hexane, décane, dodécane, le méthylal est un solvant particulièrement préféré.

En ce qui concerne les conditions opératoires, en particulier les températures et les proportions $H_2S$/oléfines, elles sont les mêmes que dans la technique correspondante, déjà connue par des publications antérieures. Il n'y a donc pas lieu de les décrire en détail ici; on notera seulement que les rapports molaires préférés $H_2S$/oléfines sont d'environ 3 à 10, la température étant maintenue entre 0 et 20°, des températures s'échelonnant de -5 à +35 étant néanmoins utilisables, bien que moins avantageuses.

En ce qui concerne la nature des composés éthyléniques, dont la transformation en mercaptan peut être améliorée par le procédé de l'invention, on peut citer, à titre d'exemples, non limitatifs: éthylène, propylène, butène-1, butène-2, isobutène, pentène-1, pentène-2, hexène-1, heptène-1, octène-1, décène-1, undécène-1, dodécène-1, tétradécène-1, hexadécène-1, octadécène-1, eicosène-1, isopentène-1, méthyl-4-pentène-1, diméthyl-3,6-eptène-1, méthyl-4-butyl-5-décène-4, diphényl-1,4-butène-2, cyclohexyl-3-eicosène-6, méthyl-4-pentène-2, triméthyl-2,4,4-pentène-2-cyclopentène, diéthyl-2,5-cyclopentène, cyclohexène, éthyl-3-cyclohexène, cyclopentène, cyclooctène, vinyl-4-cyclohexène, esters alkyliques des acides acrylique, méthacrylique, crotonique, allyl-acétique (penténoique), octylénique, undécylénique, dodécylénique, octadécylénique, etc.; les esters en question peuvent notamment contenir des alkyles en $C_1$ à $C_{30}$ et plus particulièrement en $C_1$ à $C_8$. Les composés éthyléniques peuvent renfermer plusieurs doubles liaisons, comme c'est par exemple le cas du butadiène ou de l'hexadiène-2,4.

Les exemples qui suivent, non limitatifs, illustrent l'invention.

### Exemples Comparatifs 1 à 3

Les préparations suivantes ont été effectuées selon le mode opératoire connu en soi.

Le réacteur photochimique en pyrex est du type classique à lampe plongeante coaxiale et fritté de verre à la base pour obtenir une bonne diffusion des gaz hydrogène sulfuré et propylène. Le volume irradié est de 130 ml. Ce réacteur thermostaté par une double enveloppe extérieure fonctionne en discontinu à pression ambiante, les débits gazeux étant maintenus constants au cours de l'expérience. Ils sont notamment de 30 l/heure pour $H_2S$ et de 28 l/h pour le propylène. Le solvant est l'éther diméthylique du diéthylène-glycol, et la température, à laquelle on le sature d'hydrogène sulfuré et de propylène, est de 0°C. La concentration en photosensibilisant répond à la règle du profil optimal de l'intensité absorbée dans le réacteur. La source lumineuse est une lampe à basse pression de mercure à réémission centrée sur 350 nm. Sa puissance est de 8 w. Après 35 minutes, les quantités de mercaptan formé ont été:

| Exemple | Initiateur | Mole de mercaptan |
|---|---|---|
| 1 | Benzophénone seule 0,014 mole/litre | 0,12 |
| 2 | Phosphite de tridécyle 0,060 mole/litre | 0,07 |
| 3 | Benzophénone 0,014 mole/litre conjointement avec du phosphite de tridécyle 0,060 mole/litre | 0,18 |

L'exemple 3 montre que l'emploi conjoint de la benzophénone et du phosphite donne une quantité de mercaptan 2,25 fois plus grande que ne fournit le phosphite seul.

### Exemple 4
#### Préparation du n-propane-thiol

La réaction photochimique est réalisée dans un réacteur cylindrique en pyrex de 250 ml à lampe plongeante, coaxiale. La réfrigération et l'agitation du milieu réactionnel sont assurées par une boucle extérieure refroidie de façon à maintenir la température aux alentours de 18°C. Une lampe fluorescente de 8 W, centrée sur 350 nm, équipe le réacteur.

160 ml d'éther diméthylique du diéthylène glycol (diglyme) sont introduits dans le réacteur; ce

3

# 0 005 400

solvant est ensuite saturé, sous une pression de 3 bars, avec un mélange gazeux de $H_2S$ et propylène. Le débit de l'hydrogène sulfuré est de 90 l/h, celui du propylène de 30 l/h, et ces débits sont maintenus pendant toute la durée de l'opération.

Un amortisseur de pulsations, à la sortie des gaz vers la torche, permet de maintenir une pression constante dans le réacteur. Le système est relié à une soupape de sécurité à contre pression d'azote. Une électrovanne, asservie au niveau des réactifs, permet une évacuation régulière de l'effluent que l'on récupère après passage par une enceinte de dépressurisation formant sas.

Le solvant contient par litre 0,0055 mole de benzophénone et 0,0022 mole de phosphite de triphényle; il est ajusté en continu, au moyen d'une pompe doseuse, à raison de 350 ml/l.

Lorsqu'un régime continu, stable, est atteint dans le réacteur, le volume du mélange réactionnel atteint 240 ml.

Ainsi le réacteur a produit, en continu, par heure, 20,13 g de n-propylthiol et 4,04 g de dipropyl-sulfure. Le rendement massique en mercaptan est donc de 83,3%.

Le mercaptan est récupéré par distillation après étêage de l'$H_2S$ et du propylène résiduel que l'on recycle.

## Exemple 5

Les opérations de l'exemple 4 sont répétées en injectant 120 litres à l'heure d'hydrogène sulfuré et 10 litres à l'heure de propylène. La production est de 21,5 g à l'heure avec un rendement de 90% en mercaptan.

## Exemple 6

Les opérations de l'exemple 1 sont répétées avec du butène-1 à la place du propylène.
On obtient alors 0,280 mole de butyl mercaptan.

## Exemple 7

Dans le mode opératoire de l'exemple 1, on remplace la benzophénone par de la thiobenzophé-none, (5, $10^{-4}MC^{-1}$), toutes les autres conditions étant inchangées. La quantité de mercaptan formée est de 0,150 M.

## Exemple 8

Dans les opérations de l'exemple 1, l'éther diméthylique du diéthylène glycol est remplacé par le solvant constitué par du dodécane.
Le rendement en mercaptan est de 24% pour 0,172 mole de mercaptan + sulfure formés.

## Exemple 9

Dans les opérations de l'exemple 1, l'éther diméthylique est remplacé par du méthylal.
La quantité de mercaptan formé est de 0,268 mole.

## Exemple 10

Dans les conditions de l'exemple 3, on injecte dans le milieu réactionnel de l'hydrogène sulfuré à un débit de 38 litres à l'heure. On irradie ainsi 153 mmoles d'octène-1 dissous dans du diglyme à raison de 1,274 mole par litre, contenant les mêmes initiateurs photo radicalaires que dans l'exemple 3. La température est de 1°C. Après 5 mn on a formé 81 mmoles de n-octyl mercaptan.

## Exemple 11

Dans les conditions de l'exemple 3, avec un débit de 38 l par heure d'hydrogène sulfuré on irradie 67,7 mmoles de dodécène-1 dissous dans du diglyme à la concentration de 0,564 mole par litre, à 11°C. Après 3 mn d'irradiation on a formé 48,2 mmoles de n-dodécyl mercaptan.

## Exemple 12

Préparation du mercapto 11 undecanoate de méthyle. Dans l'appareil de l'exemple 4 on injecte une solution de 200 ml d'undécylénate de méthyle dans 660 ml de diglyme renfermant, par litre, 2 g de benzophénone et 1,8 g de triphénylphosphite; cette injection est effectuée encontinu, sous une pression de 5 bars, à raison de 200 ml par heure.

Le débit de $H_2S$, qui traverse le milieu réactionnel, est de 120 l/h. L'opération a lieu à 15°C. On extrait de l'effluent 94 g de mercapto-undécanoate de méthyle et 3 g de sulfure symétrique correspondant; la sélectivité en mercaptan est d'environ 97%.

## Revendications

1. Procédé de synthèse d'un mercaptan par réaction photochimique d'un composé éthylénique avec de l'hydrogène sulfuré, en présence d'un promoteur constitué par un composé organique d'un élément du groupe V A de la Classification Périodique des Eléments, caractérisé en ce que le milieu réactionnel contient en outre une benzophénone ou/et une thiobenzophénone ces adjuvants pouvant

4

porter des substituants dans un ou les deux noyaux benzéniques.

2. Procédé suivant la revendication 1, caractérisé en ce que la benzophénone ou la thiobenzophénone porte un ou plusieurs substituants alkyle, hydroxyle, halogène, carboxyle, alkoxy ou ester dans le noyau benzénique.

3. Procédé suivant la revendication 1 ou 2, caractérisé en ce que la lumière utilisée a une longueur d'onde dans la marge de 300 à 400 nm, l'adjuvant étant une benzophénone.

4. Procédé suivant la revendication 1 ou 2, caractérisé en ce que la lumière utilisée a une longueur d'onde dans la marge de 350 à 600 nm, l'adjuvant étant une thiobenzophénone.

5. Procédé suivant une des revendications 1 et 2, caractérisé en ce que la lumière utilisée présente plusieurs longueurs d'onde entre 300 et 600 nm, et l'adjuvant utilisé est un mélange de benzophénone et de thiobenzophénone.

6. Procédé suivant une des revendications 1 à 5, caractérisé en ce que le milieu réactionnel contient 0,001 à 0,05, et de préférence 0,0015 à 0,0075 mole de benzophénone ou/et thiobenzophénone par litre.

7. Procédé suivant une des revendications 1 à 6, caractérisé en ce que la réaction a lieu dans un éther de l'éthylène glycol, dans un hydrocarbure aromatique ou aliphatique ou dans du méthylal comme solvants.

## Claims

1. Process of synthesis of a mercaptan by photochemical reaction of an ethylenic compound with hydrogen sulphide in the presence of a promoter constituted by an organic compound of an element of Group Va of the Periodic Classification of the Elements, characterised in that the reaction medium also contains a benzophenone and/or a thiobenzophenone, these additives optionally carrying substituents on one or both of the benzene rings.

2. Process according to claim 1, characterised in that the benzophenone or thiobenzophenone carries one or more alkyl, hydroxyl, halogen, carboxyl, alkoxy or ester substituents on the benzene ring.

3. Process according to claim 1 or 2, characterised in that the light utilized has a wavelength in the range from 300 to 400 nm, the additive being a benzophenone.

4. Process according to claim 1 or 2, characterised in that the light utilized has a wavelength in the range from 350 to 600 nm, the additive being a thiobenzophenone.

5. Process according to either of claims 1 and 2, characterised in that the light utilized has several wavelengths between 300 and 600 nm and the additive utilized is a mixture of benzophenone and thiobenzophenone.

6. Process according to any of claims 1 to 5, characterised in that the reaction medium contains 0.001 to 0.05 and, preferably, 0.0015 to 0.0075 mole of benzophenone and/or thiobenzophenone per litre.

7. Process according to any of claims 1 to 6, characterised in that the reaction takes place in an ethylene glycol ether, in an aromatic or aliphatic hydrocarbon or in methylal as solvents.

## Patentansprüche

1. Verfahren zur Synthese eines Mercaptans durch photochemische Reaktion einer äthylenischen Verbindung mit Schwefelwasserstoff in Anwesenheit eines Promotors, der aus einer organischen Verbindung eines Elements der Gruppe V A des Periodensystems der Elemente gebildet ist, dadurch gekennzeichnet, dass das Reaktionsmedium ausserdem ein Benzophenon und/oder ein Thiobenzophenon enthält, wobei diese Zusätze Substituenten in einem oder den beiden Benzolringen tragen können.

2. Verfahren nach Anspruch 1, dadurch gekennzeichnet, dass das Benzophenon oder das Thiobenzophenon einen oder mehrere Alkyl-, Hydroxyl-, Halogen-, Carboxyl-, Alkoxy- oder Estersubstituenten in dem Benzolring trägt.

3. Verfahren nach Anspruch 1 oder 2, dadurch gekennzeichnet, dass das verwendete Licht eine Wellenlänge im Bereich von 300 bis 400 nm hat und der Zusatz ein Benzophenon ist.

4. Verfahren nach Anspruch 1 oder 2, dadurch gekennzeichnet, dass das verwendete Licht eine Wellenlänge im Bereich von 350 bis 600 nm hat und der verwendete Zusatz ein Thiobenzophenon ist.

5. Verfahren nach einem der Ansprüche 1 und 2, dadurch gekennzeichnet, dass das verwendete Licht mehrere Wellenlängen zwischen 300 und 600 nm aufweist und der verwendete Zusatz ein Gemisch von Benzophenon und Thiobenzophenon ist.

6. Verfahren nach einem der Ansprüche 1 bis 5, dadurch gekenzeichnet, dass das Reaktionsmedium 0,001 bis 0,05 und vorzugsweise 0,0015 bis 0,0075 Mol Benzophenon und/oder Thiobenzophenon pro Liter enthält.

7. Verfahren nach einem der Ansprüche 1 bis 6, dadurch gekennzeichnet, dass die Reaktion in einem Äther von Äthylenglykol, in einem aromatischen oder aliphatischen Kohlenwasserstoff, oder in Methylal stattfindet.